# EUROPEAN PATENT APPLICATION

(11) **EP 0 664 294 A1**
(43) Date of publication of application: **26.07.1995**
(21) Application number: 95300315.9
(22) Date of filing: 19.01.1995
(51) Int. Cl.: C07D 473/00, C07D 473/24, C07H 19/16

(54) **Production of nucleoside analogues**

(30) Priority: 19.01.1994 GB 9400987
(71) Applicant: Reese, Colin, London WC2R 2LS (GB)
(72) Inventor: Reese, Colin, London WC2R 2LS (GB)
(74) Representative: Sturt, Clifford Mark

(57) **Abstract**

A process for the preparation of a purine derivative corresponding to the following general formula: 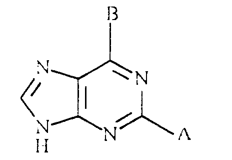
characterised in that it comprises: protecting the hydroxyl groups of the sugar moiety of a starting material corresponding to the following general formula: 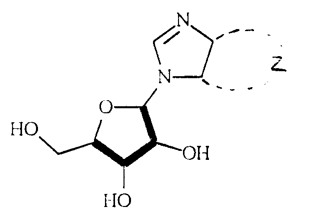
activating the 6-position of the purine ring; introducing the substituent B at this position;
and removing the sugar moiety;
wherein
A represents NH₂ or H;
B represents a substituent which has the effect of favouring subsequent substitution at the 9-position of the purine ring and which will withstand removal of the sugar moiety, but which may later be removed or converted;
and
Z represents: 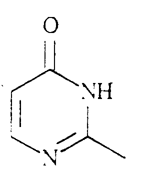
is disclosed.

The present purine derivatives obtainable by the above process may be used in a disclosed process for the preparation of a nucleoside analogue characterised in that it comprises; introducing a side chain at the 9-position of the purine derivative; and removing or converting the substituent B at the 6-position of the purine derivative.

## Description

This invention relates to the production of nucleoside analogues; more particularly, it relates to the conversion of guanosine into acyclovir, 9-[(2-hydroxyethoxy)methyl]-guanine, and the 6-deoxy derivative thereof.

In a currently-preferred first embodiment, the present invention provides a process for the preparation of a purine derivative corresponding to the following general formula: 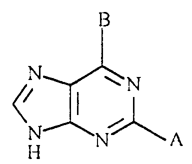
characterised in that it comprises: if necessary protecting the hydroxyl groups of the sugar moiety of a starting material corresponding to the following general formula: 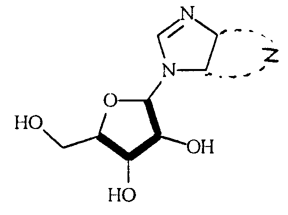
activating the 6-position of the purine ring; introducing the substituent B at this position; and removing the sugar moiety;
wherein
A represents H or a substituent such as NH₂;
B represents a substituent which has the effect of favouring subsequent substitution at the 9-position of the purine ring and which will withstand removal of the sugar moiety, but which may later be removed or transformed; and
Z represents:

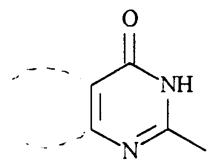

The starting material may be guanosine or inosine, for example, the hydroxyl groups of which may be protected by acylation, preferably acetylation. Generally, the activation of the 6-position involves chlorination. For example, the substituent B may be a bulky alkanethio or arenethio group, preferably an arenethio group, more preferably a group derived from p-chloro- or p-methyl-thiophenol. The sugar moiety may be removed using a Lewis acid, preferably BF₃OEt₂, or a strong protic acid, preferably c.H₂SO₄.

The present invention also provides such purine derivatives as are obtainable by this process.

In a currently-preferred further embodiment, the present invention provides a process for the preparation of a nucleoside analogue characterised in that it comprises: introducing a side chain at the 9-position of the purine derivative; and removing or converting the substituent B at the 6-position of the purine derivative.

The nucleoside analogue prepared may, for example be acyclovir, 6-deoxyacyclovir, ganciclovir, penciclovir or famciclovir.

Having indicated the general scope of the present invention, it will now be described in more detail.

A number of nucleoside analogues in which the sugar residues have been replaced by acyclic side-chains have been found to possess high antiviral activity, (see, for example, De Clercq, E., Biochem. Pharmacol., 1991, 42, 963). A particularly notable group of such analogues, which have either already found or are likely to find application in chemotherapy, are achiral 9-alkylguanine or closely related 9-alkyl-2-aminopurine derivatives. This group of compounds includes acyclovir, (see, for example, Elion, G.B., et al, Proc. Natl. Acad. Sci. USA., 1977, 74, 5716; and Schaeffer, H.J., et al, Nature, 1978, 272, 583): 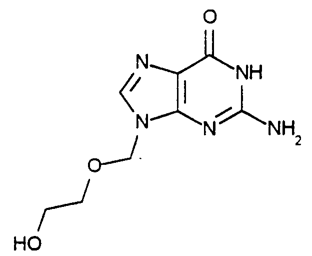
the 6-deoxy derivative thereof, (see, for example, Krenitsky, T.A., et al, Proc. Natl. Acad. Sci. USA, 1984, 81, 3209): 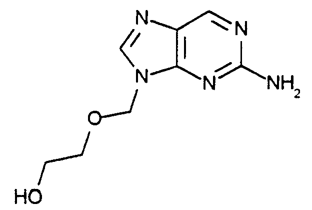
ganciclovir, 9-[(1,3-dihydroxy-2-propoxy)methyl]-guanine, (see, for example, Martin, J.C., et al, J. Med, Chem., 1983, 26, 759: Field, A.K., et al, Proc. Natl. Acad. Sci. USA., 1983, 80, 4139; Ogilvie, K.K., et al, Can. J. Chem., 1982, 60, 3005; and Schaeffer, H.J., in "Nucleosides, nucleotides and their biological-applications", Eds. Rideout, Henry & Beacham, Academic, New York, 1983, 1-17): 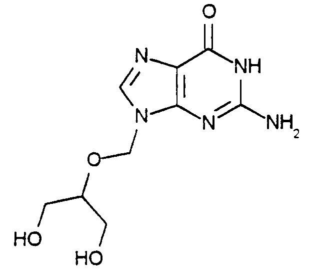
penciclovir, 9-[4-hydroxy-3-(hydroxymethyl)-but-1-yl] guanine, (see, for example, Harnden, M.R., & Jarvest, R.L., Tet. Lett., 1985, 26, 4265; and Harnden, M.R., et al, J. Med. Chem., 1987, 30, 1636): 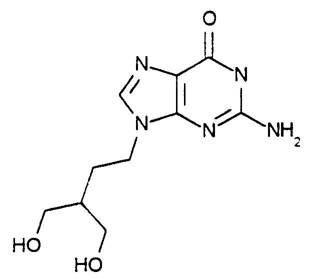
and famciclovir, 9-[4-acetoxy-3-(acetoxymethyl)-but-1-yl]-2-amino-9H-purine, (see, for example, Harnden, M.R., et al, J. Med. Chem., 1989, 32, 1738; and Geen, G.R., et al, Tet. Lett., 1992, 33, 4609):

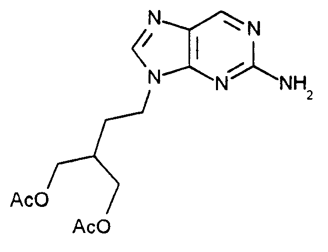

A possible overall strategy for the synthesis of these important alkylated purines consists essentially of two main parts. The first such part involves the preparation of a purine derivative that undergoes alkylation highly regioselectively (or preferably regiospecifically) on N-9, and is so designed that the resulting alkylation product may readily be converted into the corresponding 9-alkylguanine or 9-alkyl-2-aminopurine. The other main part involves the preparation of the synthons required for the introduction of the appropriate acyclic side-chains. The present process concerns particularly the first part of the overall strategy, viz the synthesis of a suitable purine derivative. In preferred embodiments, the present invention relates to the production of acyclovir and the corresponding prodrug 6-deoxy-acyclovir.

As a potential precursor of both guanine and 2-aminopurine derivatives, 2-amino-6-chloropurine, (see, for example, Davies, G. D. et al, J. Am. Chem. Soc., 1960, 82, 2633; Balsiger, R. W., & Montgomery, J. A., J. Org. Chem., 1960, 25, 1573; and Japan Patent, 1986, JP 61, 227, 583; Chem. Abstr., 1987, 106, 84280 j): 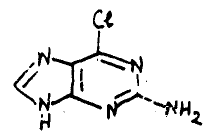
is a candidate for the purine component required in the synthesis of the above antiviral agent and indeed has been used for the purpose, (see, for example, Harnden & Jarvest, loc cit; first Harnden, et al, loc cit; second Harnden, et al, loc cit; Geen, et al, loc cit; and Robins, M. J., & Hatfield, P. W., Can.J.Chem.,1982, 60, 547). However, there is a possible disadvantage to be taken into consideration in connection with the use of 2-amino-6-chloropurine in that, although it undergoes alkylation predominantly on N-9, significant quantities of 7-alkyl derivatives are often also obtained, (see, for example, Kjellberg, J., & Johansson, N. G., Nucleosides Nucleotides, 1989, 8, 225; and Geen, G. R. et al, Tetrahedron, 1990, 46, 6903). For example, 2-amino-6-chloropurine reacts with benzyl bromide in the presence of potassium carbonate, (see, for example, Kjellberg & Johansson, loc cit) to give the isomeric 9-N- and 7-N-benzyl derivatives in the proportions of 3.5 : 1; under similar conditions it reacts with 4-acetoxy-3-(acetoxymethyl)-1-iodobutane to give, (see, for example, Geen, G. R. et al, Tetrahedron Letters, 1990, 46, 6903) mainly 9-[4-acetoxy-3-(acetoxymethyl)-but-1-yl]-2-amino-6-chloro-9H-purine; 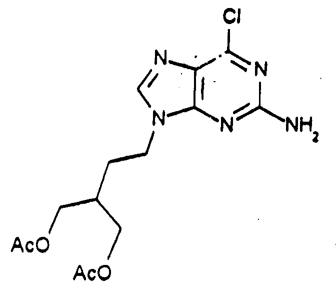
which is a precursor both of penciclovir and famciclovir, but the isomeric 7-N-alkyl derivative accounts for ca 15% of the products obtained. Since 2',3',5'-tri-O-acetylguanosine: 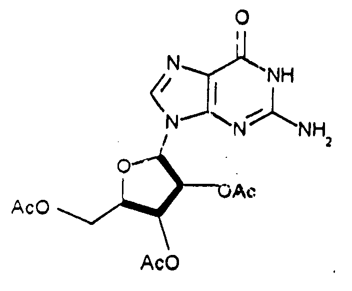
which may be prepared, (see, for example, Robins, M.J., & Uznanski, B., in "Nucleic acid chemistry. Improved and new synthetic procedures, methods and techniques, Part 3", Eds. Townsend & Tipson, Wiley, New York, 1986, 144-148), in very high (94%) yield from guanosine: 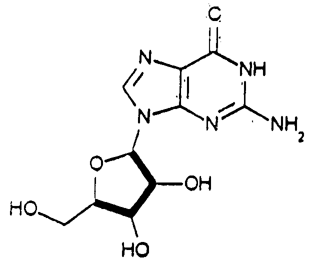
reacts with phosphorus oxychloride in the presence of N,N-dimethylaniline and tetraethylammonium chloride in acetonitrile solution to give, (see, for example Robins & Ugnanski, loc cit), 2-amino-6-chloro-9-(2,3,5-tri-O-acetyl-β-D-ribofuranosyl)purine: 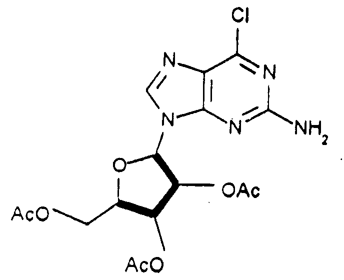
in 85 % isolated yield, it was thought that guanosine might be a more convenient starting material for the preparation of the purine component than guanine, the most accessible precursor, (Japan Patent, loc cit; Chem. Abstr., loc cit) of 2-amino-6-chloropurine. (As an alternative protecting group, benzoyl might also be considered). A recent study, (see, for example, second Geen loc cit), that the lipophilicity of the 6-substituent, as well as its bulk might influence the N-9/N-7 alkylation ratio. With this in mind, it was thought that 2-amino-6-(4-chlorophenylthio)-9H-purine: 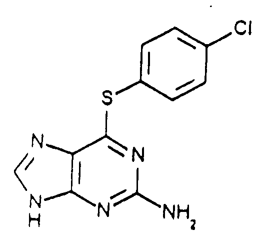
which may readily be prepared in two steps (Scheme 1 below) from the 6-chloro compound might well prove to be a suitable purine derivative for the synthesis of the above antiviral agents. 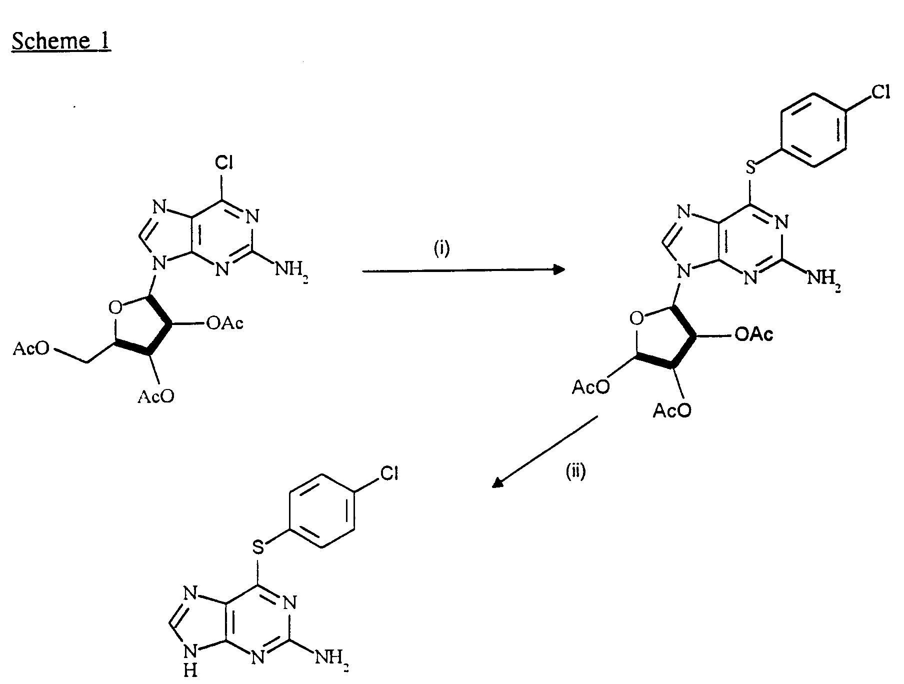
Reagents and conditions: (i) 4-ClC₆H₄SH, Et₃N, MeOH, N₂, RT, 4 h; (ii) conc. H₂SO₄, 0°C to RT or Et₂O→BF₃, PhOH, CH₂Cl₂, reflux, 2 h.

The 6-chloro-compound reacted with a slight excess each of 4-chloro(thiophenol) and triethylamine in methanol solution, (see, for example, Buck, I.M., & Reese, C.B., J.Chem. Soc., Perkin Trans. 1, 1990, 2937), at room temperature to give the corresponding thioether which was isolated as a crystalline solid in 81% yield. The key reaction in this synthetic approach is the conversion of the latter nucleoside derivative into the corresponding aglycone. Initially, this was effected using concentrated sulphuric acid at room temperature and the required purine derivative was obtained as a crystalline solid in 70% isolated yield. It was then found that a better yield (89%) was obtained when the cleavage of the glycosidic linkage was effected by treatment with an excess of boron trifluoride diethyl etherate in the presence of phenol in boiling dichloromethane solution. The presence of phenol was found to be beneficial, but it is not known whether or not it reacted with the released sugar moiety. If the glycosidic cleavage reaction is effected by the latter procedure, the four-step conversion of guanosine, which is a relatively inexpensive starting material, into the required 2-amino-6-(4-chlorophenylthio)-9H-purine proceeds in ca. 57 % overall yield.

The preparation continued according to Scheme 2: 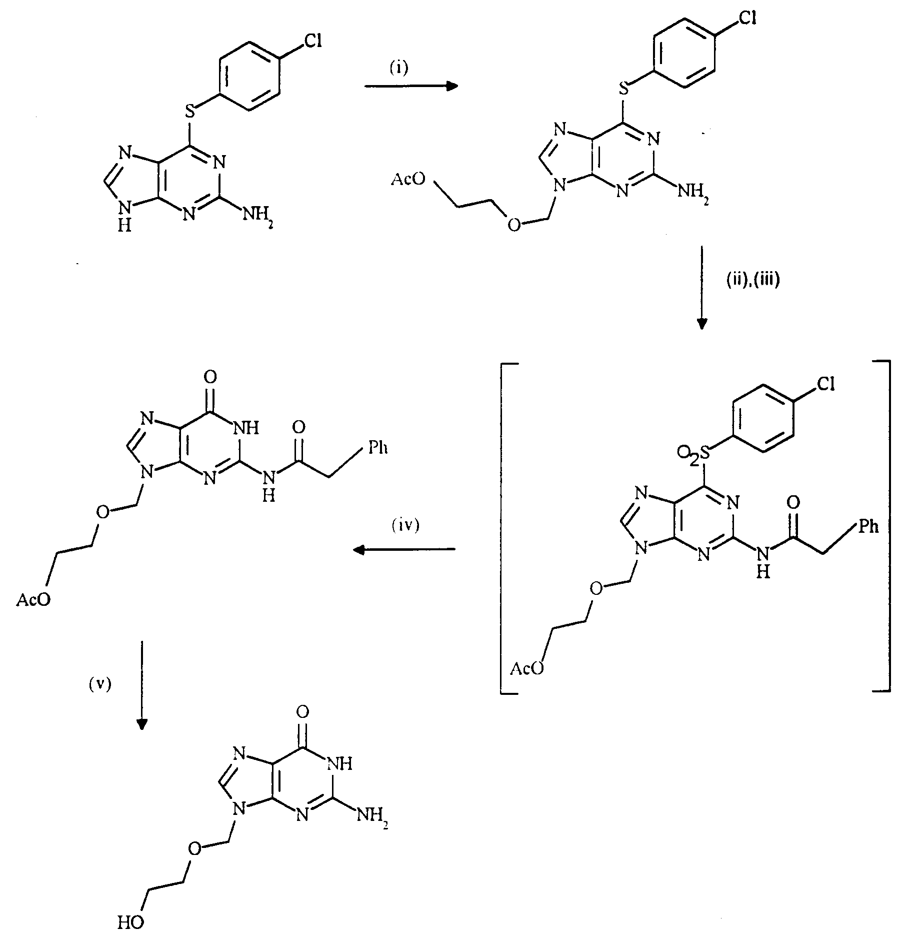
Reagents and conditions: (i), (a), (Me₃Si)₂NH, (NH₄)₂SO₄, reflux, 30 min, (b), Hg(CN)₂, AcOCH₂CH₂OCH₂Br, benzene, reflux; (ii), PhCH₂COCl, 2,6-lutidine, MeCN, 0°C; (iii), 3-ClC₆H₄CO₃H, CH₂Cl₂, RT, 3 h; (iv), Ac.C(Me)=NOH, (Me₂N)₂C=NH, MeCN, RT, 15 min; (v) 8 mol dm⁻³ NH₃, MeOH, RT, 24 h.

The purine derivative was first trimethylsilylated by treatment with an excess of hexamethyldisilazane in the presence of ammonium sulphate. The product was then heated with (2-acetoxyethoxy)methyl bromide, (see, for example, Robins & Hatfield, loc cit), and mercury (II) cyanide in benzene solution to give the 9-[(2-acetoxyethoxy)methyl] derivative in 70% isolated yield. The isomeric 7-[(2-acetoxyethoxy)methyl] derivative was not detected in the products. The compound produced was phenylacetylated on N-2 and the product was then oxidized to the sulphone by treatment with 3-chloroperbenzoic acid in dichloromethane solution, (see, for example, Buck & Reese, loc cit). The latter product was not isolated, but was treated directly with butan-2,3-dione monoxime, (see. for example, Cruickshank, K.A., PhD Thesis, London University, 1982, 87) and N¹, N¹, N³, N³-tetramethylguanidine in dry acetonitrile to give the diacylacyclovir derivative as a crystalline solid in 53% isolated yield for the three steps, starting from the thioether. The phenylacetylation step, which may at first sight appear to be superfluous, proved to be beneficial inasmuch as it improved the solubility of the intermediates in organic solvents and facilitated the isolation of a pure acyclovir derivative in satisfactory yield. 2-N-acylation of such purine systems is also believed, (see, for example, Sibanda, S., PhD Thesis, London University, 1982, 109-111) to facilitate nucleophilic attack on C-6 by oximate ions. When the diacyl derivative was treated with ammonia in methanol solution at room temperature, acyclovir was obtained and was easily isolated from the products as a pure crystalline solid in 84% yield.

While the conversion of 9-[(2-acetoxyethoxy)methyl]-2-amino-6-(4-chlorophenylthio)-9H-purine into acyclovir involved four steps, the conversion thereof into the corresponding prodrug, 6-deoxyacyclovir, (see, for example, Krenitsky, et al, loc cit), required only two steps (Scheme 3(a) below), and proceeded in almost 66% overall yield. When the thioether was heated with an excess of hydrazine hydrate in boiling ethanol solution overnight, the 6-hydrazino-compound was obtained in nearly quantitative yield. Thus smooth nucleophilic substitution by hydrazine at C-6 may readily be effected without first oxidizing the thioether to the corresponding sulphone. Treatment of the hydrazino-compound in 2-methoxyethanol solution with yellow mercury(II) oxide. (see for example, Chattopadhyaya, J.B., & Reese, C.B., J. Chem. Soc., Chem. Commun., 1977, 414), at 80°C for 2 h gave 6-deoxy-acyclovir, which was isolated as a colourless crystalline solid in 70% yield. This two-step desulphurization process was also carried out on 2-amino-6-(4-chlorophenylthio)-(2,3-O-isopropylidene-β-D-ribofuranosyl)purine. The latter compound was converted (Scheme 3(b) below) into 2-amino-(2,3-O-isopropylidene-β-D-ribofuranosyl)purine, (see, for example, Harman, R.E., et al, Chem. & Ind., 1969, 1141), in 57% overall yield. The two-step conversion (Scheme3(b) below) of 2-amino-6-(4-chlorophenyl-thio)-(2,3,5-tri-O-acetyl-β-D-ribofuranosyl)purine into the corresponding isopropylidene derivative was effected in 55% overall yield. 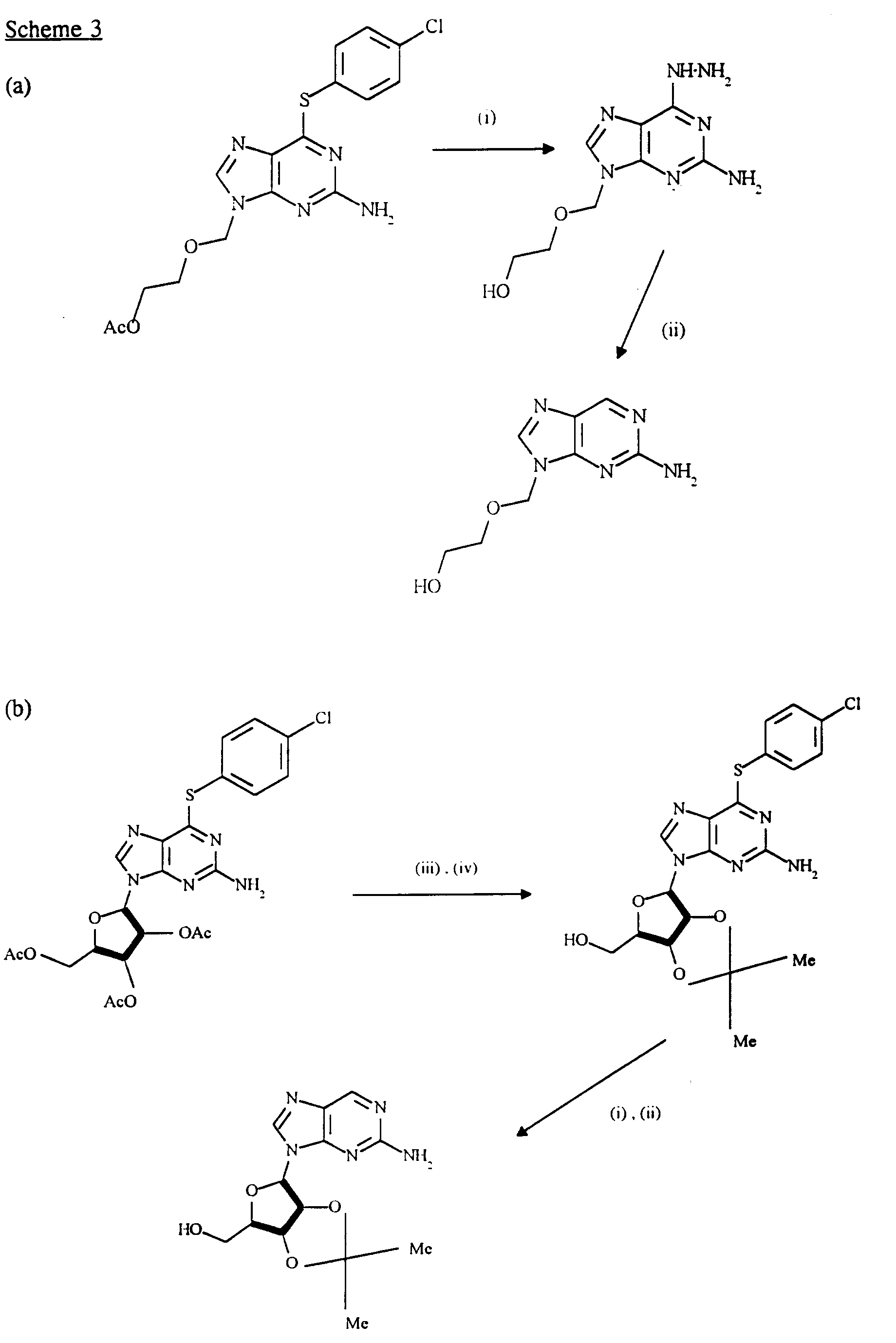
Reagents and conditions: (i) N₂H₄.H₂O, EtOH, reflux; (ii) yellow HgO, MeOCH₂CH₂OH or EtOH, ca. 80°C; (iii) 8 mol dm⁻³ NH₃, MeOH, RT, 16 h; (iv) Me₂C(OMe)₂, TsOH. H₂O, MeCN, RT.

It is thought that 2-amino-6-(4-chlorophenylthio)-9H-purine, which is best prepared by the action of boron trifluoride diethyl etherate on 2-amino-6-(4-chlorophenylthio)-(2,3,5-tri-O-acetyl-β-D-riboturanosyl)purine, will also prove to be a suitable purine derivative for the synthesis of ganciclovir, penciclovir and famciclovir.

By way of further exemplification:
NMR spectra were measured at 250 MHz with a Bruker WM 250 spectrometer and at 360 MHz with a Bruker AM 360 spectrometer; tetramethylsilane was used as an internal standard, and J-values are given in Hz. Merck silica gel 60 F2₅₄ TLC plates were developed in solvent systems A [chloroform-ethanol (98:2 v/v)], B [chloroform-ethanol (95:5 v/v)] and C [butan-1-ol - acetic acid - water (5:2:3 v/v)]. Liquid chromatography (LC) was carried out on a Jones Apex Octadecyl 5µ (micrometre) column which was eluted with 0.1 mol dm⁻³ aqueous triethylammonium acetate - acetonitrile (97:3 v/v). Dichloromethane was dried by heating over phosphorus pentoxide, under reflux, and was then distilled. Acetonitrile, 2,6-lutidine, pyridine and triethylamine were dried by heating, under reflux, with calcium hydride and were then distilled. N,N-dimethylformamide (DMF) was dried by distillation over calcium hydride under reduced (water-pump) pressure. "Light petroleum" refers to the fraction boiling in the range 30-40°C.

### 2-amino-6-chloro-(2,3,5-tri-O-acetyl-β-D-ribofuranosyl)purine:

N,N-dimethylaniline (3.2 cm³, 25.2 inmol) and freshly distilled phosphorus oxychloride (13.7 cm³, 0.147 mol) were added to a stirred solution of 2',3',5'-tri-O-acetylguanosine (10.23 g, 25.0 mmol) and tetraethylammonium chloride (8.30 g, 50.1 mmol; dried in vacuo over phosphorus pentoxide at 85°C) under an atmosphere of nitrogen at room temperature. The reaction flask was placed in an oil-bath which had been preheated to 100°C, and the reactants were heated, under reflux, for 10 min. The cooled products were then evaporated under reduced pressure, and the residue was dissolved in chloroform (150 cm³). Crushed ice (150 g) was added and the resulting mixture was stirred for 15 min. After the layers had separated, the aqueous layer was extracted with chloroform (5 x 50 cm³). The combined organic layers were then washed with cold water (6 x 30 cm³), saturated aqueous sodium hydrogen carbonate (3 x 50 cm³), dried (MgSO₄) and concentrated (to ca. 40 cm³) under reduced pressure. After propan-2-ol (60 cm³) had been added, the solution was concentrated under reduced pressure to ca. 40 cm³ and maintained at 4°C overnight to give the title compound (9.0 g, 84%) (Observed: C, 45.0; H, 3.9; N, 16.1. Calculated for C₁₆H₁₈ClN₅O₇: C, 44.9; H, 4.2; N, 16.4%) as a crystalline solid, m.p. 140-142°C, (lit 152-153°C); R_{f} 0.27 (system A); δ_{H} [(CD₃)₂SO] 2.04 (3 H, s), 2.05 (3 H, s), 2.13 (3 H, s), 4.29 (1 H, dd, J 5.1 and 10.9), 4.35-4.45 (2 H, m), 5.55 (1 H, dd, J 4.1 and 5.8), 5.89 (1 H, t, J 5.9), 6.12 (1 H, d, J 5.2), 7.09 (2 H, br.s), 8.38 (1 H, s); δ_{C} [(CD₃)₂SO] 20.12, 20.31, 20.44, 62.88, 70.18, 71.83, 79.63, 84.80, 123.43, 141.20, 149.84, 153.62, 159.84, 169.21, 169.36, 170.02.

### 2-amino-6-(4-chlorophenylthio)-(2,3,5-tri-O-acetyl-β-D-ribofuranosyl)purine

4-chloro(thiophenol) (1.70 g, 11.8 mmol) and triethylamine (1.6 cm³, 11.5 mmol) were added to a stirred suspension of 2-amino-6-chloro-(2,3,5-tri-O-acetyl-β-D-ribofuranosyl)purine (4.15 g, 9.7 mmol) in methanol under an atmosphere of nitrogen at room temperature. After 4 h, the products were filtered and the residue was washed with light petroleum. Crystallization of this material from ethanol gave the title compound (4.23 g, 81%) (Observed: C, 49.1; H, 4.0;, N, 12.9. C₂₂H₂₂ClN₅O₇S requires: C, 49.3; H, 4.1; N, 13.1%), m.p. 170°C; R_{f} 0.30 (system A); δ_{H} [(CD₃)₂SO] 2.04 (6 H, s), 2.13 (3 H, s), 4.28 (1 H, dd, J 5.5 and 11.2), 4.35 (1 H, m), 4.41 (1 H, dd J 3.7 and 11.2), 5.55 (1 H, dd, J 4.2 and 5.7), 5.89 (1 H, t, J 5.9), 6.10 (1 H, d, J 6.1), 6.57 (2 H, br.s), 7.53 (2 H, d, J 8.5), 7.64 (2 H, d, J 8.5), 8.25 (1 H, s); δ_{C} [(CD₃)₂SO] 20.19, 20.37, 20.52, 63.01, 70.31, 71.88, 79.63, 84.57, 123.66, 126.52, 129.18, 134.11, 136.63, 139.53, 151.23, 158.65, 159.70, 169.30, 169.46, 170.11.

### 2-amino-6-(4-chlorophenylthio)-9H-purine:

(a) 2-amino-6-(4-chlorophenylthio)-(2,3,5-tri-O-acetyl-β-D-ribofuranosyl)purine (3.48 g, 6.5 mmol) was added in small portions with stirring to concentrated sulphuric acid (15 cm³) at 0-5°C (ice-water bath). The reaction mixture was stirred at 0-5°C for 5 min, and then at room temperature for 30 min. The resulting solution was poured onto crushed ice (200 g), and the mixture was stirred vigorously for 5 min. (An ultrasonic bath may be used, if necessary, to effect complete solution.) The products were then carefully neutralized (to ca. pH 7) with concentrated aqueous ammonia and extracted with ethyl acetate (1 x 100cm³, 2 x 50cm³). The combined organic extracts were washed with saturated aqueous sodium hydrogen carbonate (2 x 50cm³), dried (MgSO₄) and evaporated under reduced pressure to give the title compound as a colourless solid (1.26 g, 70%) which was recrystallized from aqueous methanol (Observed: C, 47 .5; H, 2 .85; N, 25.0. C₁₁H₈ClN₅S requires: C. 47.6: H, 2.9; N, 25.2%), m.p. 225°C; R_{f} 0.05 (system A); δ_{H} [(CD₃)₂SO] 6.28 (2 H, br.s), 7.51 (2 H, d, J 8.5), 7.63 (2 H, d, J 8.5), 7.97 (1 H, s), 12.62 (1 H, br.s); δ_{C} [(CD₃)₂SO] 123.39, 127.08, 128.97, 133.71, 136.35, 139.42, 152.27, 157.01, 159.63.
(b) Phenol (2.076 g, 22 mmol) and boron trifluoride diethyl etherate (11 cm³, 89 mmol) were added to a stirred solution of 2-amino-6-(4-chlorophenylthio)-(2,3,5-tri-O-acetyl-β-D-ribofuranosyl)purine (5.91 g, 11.0 mmol) in dry dichloromethane (100 cm³), and the resulting solution was heated, under reflux, for 2 h. The cooled products were evaporated under reduced pressure. The residue obtained was dissolved in ethyl acetate (250 cm³) and the resulting solution was washed with saturated aqueous sodium carbonate (3 x 50 cm³), dried (MgSO₄) and evaporated under reduced pressure. The residue was washed with diethyl ether (3 x 20 cm³) to give the title compound as a colourless solid (2.735 g, 89%) that was identical [m.p., TLC (system B), ¹H and ¹³C NMR] to the material described in (a) above.

### 9-[(2-acetoxyethoxy)methyl]-2-amino-6-(4-chlorophenylthio)-9H-purine:

2-amino-6-(4-chlorophenylthio)-9H-purine (1.11 g, 4.0 mmol), ammonium sulphate (0.18 g, 1.36 mmol) and hexamethyldisilazane (20 cm³) were heated together, under reflux. After 3 hr, the cooled products were evaporated to dryness under reduced pressure, and dry benzene (45 cm³) and mercury(II) cyanide (1.38 g, 5.46 mmol) were added. The mixture was heated, under reflux, for 30 min and a solution of (2-acetoxyethoxy)methyl bromide (0.78 g, 3.96 mmol) in benzene (10 cm³) was added. After the reaction mixture had been heated, under reflux, for a further period of 2 h, the cooled products were evaporated under reduced pressure and chloroform (300 cm³) was added. The resulting solution was extracted with 1.0 mol dm⁻³ aqueous potassium iodide (150 cm³) and saturated aqueous sodium hydrogen carbonate (2 x 100 cm³). The dried (MgSO₄) organic layer was concentrated under reduced pressure and the residue was fractionated by chromatography on silica gel. The appropriate fractions, eluted with chloroform, were combined and evaporated under reduced pressure to give the title compound as a colourless glass (1.10 g, 70%), which was crystallized from ethyl acetate - cyclohexane (Observed: C, 48.75; H. 3.9; N, 17.4. C₁₆H₁₆ClN₅O₃S requires: C, 48.8; H, 4.1; N, 17.8%), m.p. 99-101°C; R_{f} 0.26 (system A); δ_{H} [(CD₃)₂SO] 1.95 (3 H, s), 3.69 (2 H, m), 4.07 (2 H, m), 5.45 (2 H, s), 6.50 (2 H, br.s), 7.52 (2 H, d, J 8.5), 7.63 (2 H, d, J 8.5), 8.15 (1 H, s); δ_{C} [(CD₃)₂SO] 20.46, 62.68, 66.61, 71.78, 123.36, 126.67, 129.05, 133.90, 136.45, 141.44, 151.66, 157.97. 159.87, 170.16.

### 9-[(2-acetoxyethoxy)methyl]-2-N-phenylacetylguanine:

2,6-lutidine (1.0 cm³, 8.6 mmol) was added to a stirred solution of 9-[2-(acetoxyethoxy)-methyl]-2-amino-6-(4-chlorophenylthio)-9H-purine (1.10g, 2.8 mmol) in dry acetonitrile (30 cm³), and the solution was cooled to 0°C (ice-bath). Phenylacetyl chloride (0.56 cm³, 4.2 mmol) was added, followed, after 30 min, by water (0.8 cm³). After a further period of 10 min, the products were evaporated under reduced pressure. A solution of the residue in chloroform (100 cm³) was washed with cold 1.0 mol dm⁻³ sulphuric acid (50 cm³) and then with saturated sodium hydrogen carbonate (2 x 50 cm³). The latter washings were back extracted with chloroform (50 cm³), and the combined organic extracts were dried (MgSO₄) and concentrated under reduced pressure. The residue obtained was fractionated by chromatography on silica gel. The appropriate fractions, eluted with chloroform were evaporated under reduced pressure to give a colourless glass (1.18 g), R_{f} 0.29 (system A), 0.40 (system B). 3-chloroperbenzoic acid (ca. 55%; 2.0 g, ca. 6.4 mmol) was added to a stirred solution of the latter material (1.10 g) in dichloromethane (50 cm³) at room temperature. After 3 h, more dichloromethane (50 cm³) was added and the products were washed with aqueous sodium hydrogen sulphite (50 cm³) and saturated aqueous sodium hydrogen carbonate (2 x 50 cm³). The dried (MgSO₄) organic layer was concentrated under reduced pressure and the residue was redissolved in dry acetonitrile (15 cm³). Butan-2,3-dione monoxime (0.33 g, 3.3 mmol) and N¹,N¹,N³,N³-tetramethylguanidine (0.40 cm³, 3.2 mmol) were added, and the reactants were stirred at room temperature. After 15 min, the products were concentrated under reduced pressure, the residue was redissoived in chloroform (50 cm³) and the resulting solution was washed with saturated aqueous sodium hydrogen carbonate (2 x 50 cm³). The dried (MgSO₄) chloroform layer was evaporated under reduced pressure and the residue was fractionated by chromatography on silica gel. The appropriate fractions, eluted with chloroform-ethanol (96:4 v/v), were combined and evaporated under reduced pressure to give a colourless glass. Crystallization of this material from propan-2-ol gave the title compound (0.53 g, 53% overall yield based on the purine) (Observed: C, 56.1; H, 4.9; N, 17.9. C₁₈H₁₉N₅O₅ requires: C, 56.1; H, 5.0; N, 18.2%), m.p. 148-150°C; R_{f} 0.05 (system A), 0.15 (system B); δ_{H} [(CD₃)₂SO] 1.95 (3 H, s), 3.70 (2 H, m), 3.82 (2 H, s), 4.09 (2 H, m), 5.50 (2 H, s), 7.25 - 7.40 (5 H, m), 8.16 (1 H, s), 12.03 (2 H, br.); δ_{C} [(CD₃)₂SO] 20.47, 42.49, 62.61, 66.55, 72.37, 120.23, 126.92, 128.37, 129.30, 134.20, 140.07, 148.05, 148.74, 154.79, 170.18, 174.09.

### 9-[(2-hydroxyethoxy)methyl]-guanine (Acyclovir):

9-[(2-acetoxyethoxy)methyl]-2-N-phenylacetylguanine (0.53 g, 1.38 mmol) was dissolved in 8 mol dm⁻³ methanolic ammonia (20 cm³) at room temperature. After 24 h, the products were evaporated under reduced pressure and the residue was crystallized from aqueous ethanol to give the title compound (0.26 g, 84%) (Observed: C, 42.45; H, 5.0; N, 30.8. Calculated for C₈H₁₁N₃O₃: C, 42.7; H, 4.9; N, 31.1%) as colourless crystals, m.p. 255-260°C (lit m.p. 265-266°C); λₘₐₓ (0.1 mol dm⁻³ hydrochloric acid)/nm 254 (ε 11 800); λ_{infl}/nm (ε 7800); λₘᵢₙ/nm 226 (ε 2400); R_{f} 0.25 (system C); t_{R} 4.6 min (100%); δ_{H} [(CD₃)₂SO] 3.48 (4 H, m), 4.70 (1 H, m), 5.36 (2 H, s), 6.54 (2 H, br.s), 7.84 (1 H, s), 10.69(1 H, br.s); δ_{C} [(CD₃)₂SO] 59.79, 70.26, 71.92, 116.34, 137.69, 151.32, 153.75, 156.73. (This material was identical to that purchased from the Sigma Chemical Co.)

### 3-amino-6-hydrazino-9-[(2-hydroxyethoxy)methyl]-9H-purine:

Hydrazine monohydrate (1.40 cm³, 28.9 mmol) was added to a stirred solution of 9-[(2-acetoxyethoxy)methyl]-2-amino-6-(4-chlorophenylthio)-9H-purine (1.345 g, 3.4 mmol) in absolute ethanol (25 cm³). The resulting solution was heated, under reflux, for 18 h and then cooled. The products were filtered and the filtrate was evaporated under reduced pressure. The residue was triturated with diethyl ether (3 x 30 cm³) to give the title compound (0.77 g, 94%) (Observed in material recrystallized from absolute ethanol: C, 40.6; H, 5.3; N, 40.8. C₈H₁₃N₇O₂ requires: C, 40.2; H, 5.5; N, 41.0%), m.p. 178-180°C; R_{f} 0.19 (system B); δ_{H} [(CD₃)₂SO] 3.47 (4 H, s), 4.45 (2 H, br), 4.72 (1 H, m), 5.39 (2 H, s), 6.05 (2 H. s), 7.85 (1 H, s), 8.58 (1 H, br.s); δ_{C} [(CD₃)₂SO] 59.81, 70.18, 71.64, 111.73, 137.46, 151.34, 155.83, 160.29.

### 2-amino-9-[(2-hydroxyethoxy)methyl]-9H-purine:

Yellow mercury(II) oxide (2.18 g, 10.1 mmol) was added to a stirred solution of 2-amino-6-hydrazino-9-[(2-hydroxyethoxy)methyl]-9H-purine (0.80 g, 3.3 mmol) in dry 2-methoxyethanol (60 cm³) and the resulting suspension was heated at 80°C for 2 h. The products were filtered and the filtrate was concentrated under reduced pressure. The residue was fractionated by short column chromatography on silica gel: the appropriate fractions, which were eluted with chloroform-methanol (95:5 v/v), were combined and evaporated under reduced pressure to give 2-amino-9-[(2-hydroxyethoxy)methyl]-9H-purine as a colourless solid (0.49 g, 70%) (Observed in material crystallized from absolute ethanol: C, 46.1; H, 5.4; N, 33.4. Calculated for C₈H₁₁N₅O₂: C, 45.9; H, 5.3; N, 33.5%), m.p. 185°C (lit. 187-189°C); R_{f} 0.11 (system B); δ_{H} [(CD₃)₂SO] 3.49 (4 H, m), 4.70 (1 H, t, J 5.3), 5.48 (2 H, s), 6.62 (2 H, s), 8.20 (1 H, s), 8.62 (1 H, s); δ_{C} [(CD₃)₂SO] 59.79, 70.49, 71.67, 126.54, 142.77, 149.16, 153.08, 160.69.

### 2-amino-6-(4-chlorophenylthio)-(2.3-O-isopropylidene-β-D-ribofuranosyl)-purine:

2-amino-6-(4-chlorophenylthio)-(2,3,5-tri-O-acetyl-β-D-ribofuranosyl)purine (5.50 g, 10.26 mmol) and methanolic ammonia (8 mol dm⁻³, 100 cm³) were stirred together at room temperature. After 16 h, the products were concentrated under reduced pressure to give a colourless solid residue. 2,2-dimethoxypropane (12.0 cm³, 95.5 mmol) and toluene-4-sulphonic acid monohydrate (1.97 g, 10.36 mmol) were added to a stirred solution of the latter material in acetonitrile (50 cm³) at room temperature. After 30 min, the products were neutralized (pH paper) with methanolic ammonia, and then filtered. The filtrate was evaporated under reduced pressure and the residue was fractionated by short column chromatography on silica gel: the appropriate fractions, which were eluted with dichloromethane-methanol (99:1 v/v), were combined and concentrated under reduced pressure to give the title compound as a colourless glass (2.56 g, 55%) (Observed in material crystallized from absolute ethanol: C, 50.8; H, 4.6; N, 15.4. C₁₉H₂₀ClN₅O₄S requires: C, 50.8; H, 4.5; N, 15.6%), m.p. 205-207°C; R_{f} 0.26 (system A); δ_{H} [(CD₃)₂SO] 1.32 (3 H, s), 1.52 (3 H, s), 3.52 (2 H, m), 4.15 (1 H, m), 5.03 (2 H, m), 5.28 (1 H, dd, J 2.4 and 6.2), 6.03 (1 H, d, J 2.4), 6.51 (2 H, br.s), 7.51 (2 H, dd, J 1.9 and 6.6), 7.62 (2 H, dd, J 1.9 and 6.6), 8.20 (1 H, s); δ_{C} [(CD₃)₂SO] 25.23, 27.02, 61.56, 81.27, 83.43, 86.99, 88.65, 112.94, 123.69, 126.71, 129.13, 133.99, 136.52, 139.78, 150.96, 158.21, 159.59.

### 2-amino-(2,3-O-isopropylidene-β-D-ribofuranosyl)purine:

Hydrazine monohydrate was added to a solution of 2-amino-6-(4-chlorophenylthio)(2,3-0-isopropylidene-β-D-ribofuranosyl)purine (0.50 g, 1.1 mmol) in absolute ethanol (10 cm³). The resulting solution was heated, under reflux for 6 h, and the products were cooled and evaporated under reduced pressure. The residue was triturated with diethyl ether (3 x 10 cm³) and then dissolved in absolute ethanol (15 cm³). Yellow mercury(II) oxide (0.72 g, 3.3 mmol) was added and the stirred suspension was heated, under reflux, for 1 h. The cooled products were filtered through Celite, and the filtrate was concentrated under reduced pressure. The residue was fractionated by short column chromatography on silica gel: the appropriate fractions, which were eluted with chloroform-ethanol ( 98 : 2 v/v), were combined and evaporated under reduced pressure to give the title compound as a colourless glass (0.196 g, 57%) (Observed in material crystallized from ethyl acetate - cyclohexane: C, 50.8; H, 5.8; N, 22.45. Calculated for C₁₃H₁₇N₅O₄: C, 50.8; H, 5.6; N, 22.8%), m.p. 118-120°C; R_{f} 0.25 (system B); δ_{H} [(CD₃)₂SO] 1.33 (3 H, s), 1.54 (3 H, s), 3.54 (2 H, m), 4.17 (1 H, m), 5.03 (1 H, dd, J 2.9 and 6.2), 5.07 (1 H, t, J 5.2), 5.32 (1 H, dd, J 2.6 and 6.2), 6.08 (1 H, d, J 2.6), 6.64 (2 H, br.s), 8.28 (1 H, s), 8.61 (1 H, s); δ_{C} [(CD₃)₂SO] 25.15, 26.97, 61.49, 81.19, 83.29, 86.74, 88.32, 112.91, 126.81, 140.96, 149.36, 152.36, 160.39.

Presently-preferred embodiments of the present invention may be summarized as follows:
2-amino-6-(4-chlorophenylthio)-(2,3,5-tri-O-acetyl-β-D-ribofuranosyl)-purine,whichis readily prepared by allowing the corresponding 6-chloro-compound to react with 4-chloro(thiophenol) and triethylamine in methanol solution at room temperature, reacts with boron trifluoride diethyl etherate in boiling dichloromethane solution to give 2-amino-6-(4-chlorophenylthio)-9H-purine in high isolated yield. 9-[(2-Acetoxyethoxy)methyl]-2-amino-6-(4-chlorophenylthio)-9H-purine, prepared from the latter aglycone in good yield, is converted by a four-step process into acyclovir and by a two-step process into 6-deoxy-acyclovir.

Having illustrated the present process fairly specifically, it would seem appropriate finally to summarize it in more general terms. The contemplated first step involves the protection of the hydroxyl groups of the sugar moiety by acylation, preferably acetylation, so that the groups are protected as esters. Secondly, the 6-position of the purine is activated, e.g. by chlorination, and then a bulky thiolate salt, such as a salt of an aromatic thiol (e.g. p-chloro or p-methyl-thiophenol), is introduced at this position. This substituent will be required to be displaced later, but it must survive the removal of the sugar moiety, which is the next step. The sugar moiety is preferably removed using a Lewis acid, such as BF₃OEt₂, or a strong protic acid, such as c.H₂SO₄. Thus, an intermediate, which could also be prepared from a suitable purine derivative, such as 2-amino-6-chloro-9H-purine, is produced. Thereafter, further conversion may be effected by generally conventional means to introduce a desired side-chain on the 9-position. Lastly, the 6-substituent may be removed (i.e. replaced by H) or converted to =O depending upon whether a 6-deoxy or a guanosine derivative is required.

A similar approach might be applied to an inosine starting material, for example, in order to obtain 9-alkyladenine or 9-alkylpurine derivatives.

## Claims

1. A process for the preparation of a purine derivative corresponding to the following general formula: 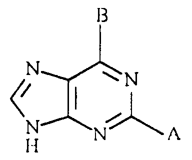 characterised in that it comprises: if necessary protecting the hydroxyl groups of the sugar moiety of a starting material corresponding to the following general formula: 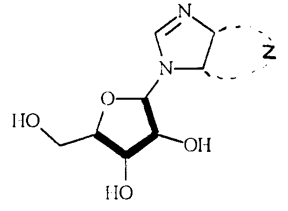 activating the 6-position of the purine ring; introducing the substituent B at this position; and removing the sugar moiety;
wherein
A represents H or a substituent such as NH₂;
B represents a substituent which has the effect of favouring subsequent substitution at the 9-position of the purine ring and which will withstand removal of the sugar moiety, but which may later be removed or transformed; and
Z represents:

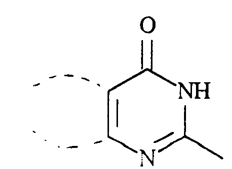

2. A process as claimed in claim 1 wherein the starting material is guanosine or inosine.

3. A process as claimed in claim 1 or claim 2 wherein the hydroxyl groups are protected by acylation, preferably acetylation.

4. A process as claimed in any of claims 1 to 3 wherein the activation is by chlorination.

5. A process as claimed in any of claims 1 to 4 wherein the substituent B is a bulky alkanethio or arenethio group, preferably an arenethio group, more preferably a group derived from p-chloro- or p-methyl-thiophenol.

6. A process as claimed in any of claims 1 to 5 wherein the sugar moiety is removed using a Lewis acid, preferably BF₃OEt₂, or a strong protic acid, preferably c.H₂SO₄.

7. A purine derivative characterised in that it corresponds to the following general formula: 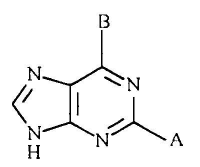 wherein A and B are as indicated above.

8. A process for the preparation of a nucleoside analogue characterised in that it comprises: introducing a side chain at the 9-position of a purine derivative prepared by a process as claimed in any of claims 1 to 6 or as claimed in claim 7; and removing or converting the substituent B at the 6-position of the purine derivative.

9. A process as claimed in claim 8 wherein the nucleoside analogue prepared is acyclovir, 6-deoxyacyclovir, ganciclovir, penciclovir or famciclovir.

10. The invention substantially as herein described with particular reference to the exemplification.
